(19) Europäisches Patentamt
European Patent Office
Office européen des brevets

(11) EP 2 829 536 A1

(12) EUROPEAN PATENT APPLICATION

(43) Date of publication:
**28.01.2015 Bulletin 2015/05**

(51) Int Cl.:
***C07D 231/46*** *(2006.01)* ***A61K 31/4152*** *(2006.01)*
***A61P 31/06*** *(2006.01)*

(21) Application number: **13178220.3**

(22) Date of filing: **26.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **LIONEX Diagnostics and Therapeutics GmbH**
**38126 Braunschweig (DE)**

(72) Inventors:
- **Singh, Mahavir**
  **38124 Braunschweig (DE)**
- **Kaufmann, Dieter**
  **38640 Goslar (DE)**
- **Zapolskii, Viktor**
  **38678 Clausthal-Zellerfeld (DE)**
- **Oehlmann, Wulf**
  **38126 Braunschweig (DE)**

(74) Representative: **Kröncke, Rolf**
**Gramm, Lins & Partner**
**Patent- und Rechtsanwaltssozietät GbR**
**Freundallee 13a**
**30173 Hannover (DE)**

(54) **4-Nitro-5-dichloromethylpyrazol derivatives for the treatment of infectious diseases**

(57) The present invention relates to new 4-nitro-5-dichloromethylpyrazol derivatives. These new derivatives are particularly useful in the treatment of infectious diseases including bacterial infection. In particular, the compounds according to the present invention are useful in the treatment of mycobacterial infections including tuberculosis.

EP 2 829 536 A1

## Description

The present invention relates to new 4-nitro-5-dichloro-methylpyrazolderivates. These new derivatives are particularly useful in the treatment of infectious diseases including bacterial infection. In particular, the compounds according to the present invention are useful in the treatment of mycobacterial infections including tuberculosis.

Prior art

Tuberculosis (TB) is a highly infectious air borne disease and it stays one of the major contagious determinants of morbidity and mortality worldwide. According to the world health organization estimates, around one third of world's population are infected with Mycobacterium tuberculosis (M. tb) (WHO: Tuberculosis facts, 2007). Annually, around 9 million people develop the active form of the disease with subsequent nearly 2 million deaths (Geraldes Santos et al., 2007).

The living attenuated Bacille Calmette-Guérin (BCG) vaccine is the only currently used vaccine, its widespread use is controversial. It could be valuable in preventing the less frequent severe forms of TB in young children such as meningitis and systemic disease. On the other hand, it was unable to decrease the TB pandemic. In addition, recent data suggested its contribution in the development of even more aggressive forms (Marriner et al., 2011).

The global burden of tuberculosis was exacerbated due to the co-infection with human immunodeficiency virus (HIV). HIV positive individuals have a 60-fold greater risk to develop active TB and higher rates to relapse after treatment. Furthermore, the treatment of the dual infection is problematic due to the undesirable interaction between antiviral and anti-TB drugs. Certainly the first line anti-TB agent rifampicin (RIF), which is a powerful enzyme inducer, accelerates the metabolism of several antiviral drugs (Corbett et al., 2003).

Each year, around half million individuals develop multi-drug resistant TB (MDR-TB), which means resistance to at least two of the main first-line anti-TB antibiotics, isoniazid (INH) and RIF. The extensively drug-resistant TB (XDR-TB) had emerged in 2006, which is not only resistant to first-line agents but also to allfluoroquinolones and at least one of the injectable drugs; kanamycin, amikacin and capreomycin. The estimated cure rates with MDR-TB and XDR-TB infection ranges between 60-80% and 30-60 % respectively, in comparison to 95% when the patient is infected with fully susceptible strains (Haydel, 2010). The appearance of TB which is resistant to all known drugs with increasing the number of the diagnosed cases in India emphasizes the inevitable need to find new anti-TB drugs (Rowland, 2012).

It is obvious that, the performance of the current means for TB diagnosis and treatment with its lengthy combined regimens hinders the competent implementation of the DOTS strategy which actually covers not more than 25% of all patients worldwide (Balganesh et al., 2004). The rationale of combination therapy is to prevent the development of drug resistance and due to the different contribution of each drug to target a certain population of the microbe in its different states. The lengthy treatment is needed to minimize the risk of relapses produced by the persisters, the bacilli that stay in the host in a dormant state for long periods despite appropriate drug treatment (Fenhalls et al., 2002).

For drug development, High-throughput screening has two approaches: Whole cell screens (phenotypic screening): test the effect of the substances on whole bacterial cell growth to derive 'hits' that can kill the pathogen. Several whole cell screens were established like resazurine microtitre assay (Palomino et al. 2002), luciferase-reporter mycobacterial Strain (Lee et al., 2003) and green fluorescent protein based screening systems (Changsen et al., 2003). Such screens have the disadvantage of selecting compounds with generalized toxicity (Sacchettini et al., 2008). Also the unidentified target is considered as a rate limiting step in the lead optimization process (Nuermberger et al., 2010). Cell-free target-based screens: predict the inhibitory effect of different compounds on a specific target in cell-free conditions. Different assays were designed based on the action of purified drug targets (e.g. Agren et al., 2008). The physiochemical properties and the pharmacokinetic (PK) profiles of the selected inhibitors should be evaluated to confirm permeability and whole-cell potency (Sacchettini et al., 2008). Combining phenotypic and target-based screens might help to avoid the drawbacks of each.

After lead identification, measuring the PK profiles is very important to provide an idea about the relationship between the in vitro and in vivo activities. Estimating the therapeutic window, by calculating the ratio of the toxic dose to the effective dose, is needed for reaching an acceptable safety profile. Synthetic and medicinal chemists modify the lead compounds to achieve better drug like properties and to improve in vivo efficacy (Balganesh et al., 2004).

In view of the above, there is an ongoing need for new compounds suitable for treatment of infectious diseases, in particular, treatment of bacterial infection, like mycobacterial infection including tuberculosis.

Description of the present invention

In a first aspect, the present invention relates to compounds of the general formula III

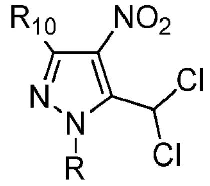

(III)

wherein
R represents in each case an optionally substituted aryl group or an optionally substituted heteroaryl group, preferably, R represents one of the radicals

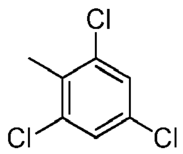

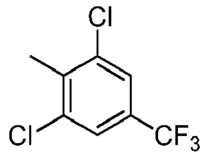

$R_{10}$ is selected from

i) $X_1$-$(C(R_{11})_n$-$X_2$-
with $X_2$ being N or O;
$X_1$ is either $R_5R_6N$- or $R_5O$-,
$R_5$ is H, or an optionally substituted, straight or branched $C_1$-$C_{10}$ alkyl group or an optionally substituted $C_3$-$C_8$ cycloalkyl group, or unsubstituted or substituted aryl, like benzyl, or an optionally substituted heteroaryl, $R_5$ is preferably one of the radicals of a methyl, ethyl or isopropyl group;
$R_6$ is H, or an acyl group (like Ac, straight or branched $C_1$-$C_{10}$ alkyl-CO, Ph-CO, Aryl-CO, Heteroaryl-CO) group, or an optionally substituted straight or branched $C_1$-$C_{10}$ alkyl group, or unsubstituted or substituted aryl group, like benzyl group, or an optionally substituted heteroaryl group;
n is an integer of from 1 to 5, preferably 1 to 3, and
$R_{11}$ is independently from each other H, or an optionally substituted straight or branched $C_1$-$C_{10}$ alkyl group, or an optionally substituted $C_3$-$C_8$ cycloalkyl group, an optionally substituted aryl group, or an optionally substituted heteroaryl group;
ii) a heterocyclic group, like a heteroaryl group, whereby said group may be substituted, and whereby said groups is linked via a heteroatom present in the heterocyclic group, like a heteroaryl group, with the pyrazol ring; or
iii) $R_{12}R_{13}N$-
with $R_{12}$ and $R_{13}$ being independently from each other H, or an optionally substituted straight or branched $C_1$-$C_{10}$ alkyl group, an optionally substituted $C_3$-$C_8$ cycloalkyl group, an optionally substituted aryl group, or an optionally substituted heterocyclic group;

or pharmaceutically acceptable salts thereof.

In particular, the present invention relates to compounds according to formula I or salts thereof according to formula II

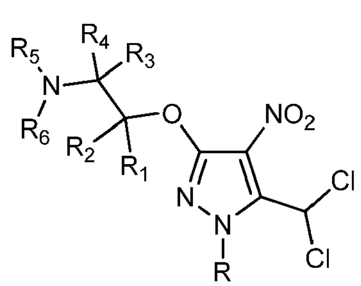

(I)

in which
R represents in each case an optionally substituted aryl group or an optionally substituted heteroaryl group.
For example, R represents in each case an optionally substituted aryl (Ph, *o*-, *m*-, *p*-$R_7C_6H_4$, di-, tri-, tetra- and penta-$R_7$-substituted aryl, like naphthyl, anthracenyl) or an optionally substituted heteroaryl (like azolyl, thienyl, pyridinyl), wherein $R_7$ is alkyl, halogen, $NO_2$, CN, $CF_3$, CHO, COOH, $CONH_2$, $SO_3H$, $SO_2NH_2$, or O-alkyl.

In a particular embodiment, R represents one of the radicals

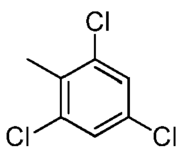

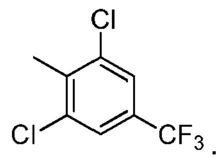

.

$R_1$-$R_4$ is independently from each other H, or an optionally substituted straight or branched $C_1$-$C_{10}$ alkyl group, or an optionally substituted $C_3$-$C_8$ cycloalkyl group, an optionally substituted aryl group, or an optionally substituted heteroaryl group, for example, $R_1$-$R_4$ is H, or a straight or branched $C_1$-$C_{10}$ alkyl group, or a $C_3$-$C_8$ cycloalkyl group, or an optionally substituted aryl group (Ph, *o*-, *m*-, *p*-$R_7C_6H_4$, di-, tri-, tetra- and penta-$R_7$-substituted aryl, like naphthyl, anthracenyl) or an optionally substituted heteroaryl group (like azolyl, thienyl, pyridinyl).
$R_5$ is H, an optionally substituted, straight or branched $C_1$-$C_{10}$ alkyl group or an optionally substituted $C_3$-$C_8$ cycloalkyl group, or unsubstituted or substituted aryl, like benzyl, or an optionally substituted heteroaryl group. $R_5$ is preferably one of the radicals of a methyl, ethyl or isopropyl group.
$R_6$ is H, or an acyl group (like Ac, straight or branched $C_1$-$C_{10}$ alkyl-CO, Ph-CO, Aryl-CO, Heteroaryl-CO) group, or an optionally substituted straight or branched $C_1$-$C_{10}$ alkyl group, or unsubstituted or substituted aryl group, like benzyl group, or an optionally substituted heteroaryl group.
The compound may be pharmaceutically acceptable salts (II) of the compound of general formula (II),

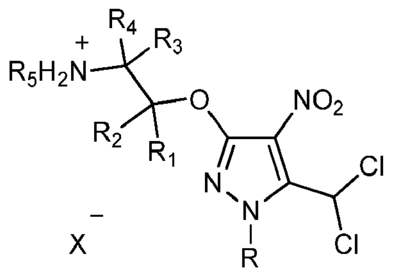

(II)

in which
$R_1$-$R_5$ are the same radicals as in formula (I)
$X^-$ is a suitable anion for forming the salt, preferably represents one of the anions $Cl^-$, $Br^-$, $BF_4^-$, $PF_6^-$ $SO_3H^-$. In addition, the compound may be present in form of its solvates or hydrates.

In another embodiment of the present invention, the $R_{10}$ is a pyrazol group, a morpholino group or a benzotriazol group which may be substituted. In addition, $R_{10}$ is a $R_{12}R_{13}N$- wherein at least one of $R_{12}$ and $R_{13}$ is not H. Preferably, the residue $R_{12}R_{13}N$- is an alkylamino group, a dialkylamino group, a cycloalkylamino group, a dicycloalkylamino group, an arylamino groor up, a heterocyclicamino group.

It has been identified by the present inventors that the compounds identified above are suitable for the treatment of infectious diseases, in particular, for the treatment of mycobacterial infection including tuberculosis.

In an advantageous embodiment, the compound according to the present invention is a compound according to formula I or salts thereof according to formula II wherein any one of $R_1$ to $R_4$ are independently selected from hydrogen or $C_1$-$C_4$ alkyl chain, in particular, wherein $R_1$ to $R_4$ are hydrogen.

In another embodiment, the compounds according to the present invention are characterized in that the residues $R_5$ and $R_6$ are independently from one another a straight or branched $C_1$-$C_4$ alkyl chain or hydrogen, in particular, at least one of the residues $R_5$ and $R_6$ is hydrogen.

Moreover, it is advantageous that the residue R of formulas I and II is a substituted aryl or heteroaryl group, like a benzyl group, in particular, wherein R is

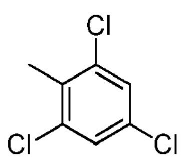

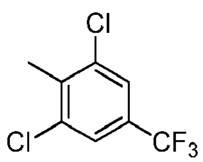

It is particularly preferred that compounds according to the present invention are compounds wherein R is

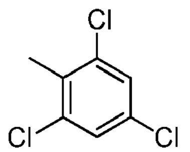 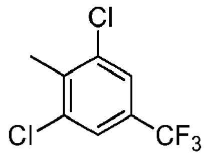,

and

$R_1$ to $R_6$ are independently from one another hydrogen and, if appropriate, $X^-$ is a halogen, like $Cl^-$ or $Br^-$.

As used herein, the term "Alkyl" refers to a saturated aliphatic hydrocarbon including straight chain and branched chain groups. "Alkyl" may be exemplified by groups such as methyl, ethyl, *n*-propyl, isopropyl, *n*-butyl and the like. Alkyl groups may be substituted or unsubstituted.

Substituents as identified herein may also be themselves substituted unless otherwise indicated. When substituted, the substituent group is preferably, but not limited to, $C_1$-$C_4$ alkyl, aryl, amino, cyano, halogen, like Cl or Br, alkoxy or hydroxyl.

As used herein, n is an integer of 1 to 5, like 1 to 3, including the embodiments of 1, 2, 3, 4, and 5.

Further, the term "$C_1$-$C_{10}$, e.g. $C_1$-$C_4$" includes all possibilities, namely, $C_1$, $C_2$, $C_3$, $C_4$, $C_5$, $C_6$, $C_7$, $C_8$, $C_9$, and $C_{10}$.

"Acyl" or "carbonyl" refers to the group -C(O)R wherein R is H, alkyl, aryl, heteroaryl, $C_1$-$C_4$ alkyl aryl or $C_1$-$C_4$ alkyl heteroaryl.

"Aryl" refers to an aromatic carbocyclic group. The term includes heteroaryl or heteroaromatic groups unless other specified. "Aryl" may be exemplified by phenyl or benzyl or naphthyl. The aryl group may be substituted or unsubstituted. Substituents may also be themselves substituted. When substituted, the substituent group of the aryl group is preferably, but not limited to, alkyl, alkoxy, heteroaryl, acyl, carboxyl, amido, carbamoyl, carbonylamino, nitro, amino, cyano, halogen or hydroxyl. The substituents may be positioned at various locations on an aryl group. For example, substituents on a phenyl group may be located at an ortho-position, a meta-position, or para-position, or combinations thereof. For example, the term "aryl" as used herein refers in particular to a phenyl group, *o*-, *m*-, *p*-$R_7C_6H_4$, di-, tri-, tetra- and penta-$R_7$-substituted aryl, naphthyl, anthracenyl, as well as to azolyl, thienyl, pyridinyl groups. In this connection, $R_7$ is alkyl, halogen, $NO_2$, CN, $CF_3$, CHO, COOH, $CONH_2$, $SO_3H$, $SO_2NH_2$, or Oalkyl.

The term "heterocyclic groups" refer to monocyclic or bicyclic carbocyclic radicals having one or more heteroatoms in the carbocyclic ring(s). The groups may be either aromatic or non-aromatic groups. Preferred, but non-limiting, heterocyclic groups are substituted or unsubstituted pyrazole, triazole, indazole, benzimidazole, benzotriazole, pyrrolidine, piperidine, piperazine, or morpholine rings. When substituted, the substituents may themselves be substituted. Preferred, but non-limiting substituents, are aryl, $C_1$-$C_4$ alkyl aryl, amino, halogen, hydroxyl, cyano, nitro, carboxyl, carbonylamino or $C_1$-$C_4$ alkyl.

"Heteroaryl" or "heteroaromatic" refers to an aromatic monocyclic or bicyclic aromatic carbocyclic radical having one or more heteroatoms in the carbocyclic ring(s). Heteroaryl may be substituted or unsubstituted. When substituted, the substituents may themselves be substituted. Preferred, but non-limiting substituents, are aryl, $C_1$-$C_4$ alkyl aryl, amino, halogen, hydroxyl, cyano, nitro, carboxyl, carbonylamino or $C_1$-$C_4$ alkyl. Preferred heteroaromatic groups include tetrazoyl, thiazolyl, thienyl, thiophenyl, thiazolyl, purinyl, pyrimidyl. pyridyl, and furanyl. More preferred heteroaromatic groups include benzothiofuranyl, thiophenyl, thienyl, furanyl, tetrazoyl, triazolyl, and pyridyl.

"Heteroatom" means an atom other than carbon in the ring of a heterocyclic group or a heteroaromatic group or the chain of a heterogeneous group. Preferably, heteroatoms are selected from the group consisting of nitrogen, sulfur, phosphor and oxygen atoms. Groups containing more than one heteroatom may contain different heteroatoms.

It has been recognized that said compounds are particularly useful in the treatment of infectious diseases, in particular, infectious diseases with microorganisms. According to the present invention, the term "infectious diseases" includes viral, bacterial, parasite or any other pathogenic organism infections.

In a preferred embodiment, the compounds according to the present invention are particularly useful in the treatment of bacterial infection. For example, the compounds according to the present invention are useful in the treatment of mycobacterial infection. As demonstrated in the examples, it is possible to treat mycobacterial infection, in particular, treating infection with mycobacterium tuberculosis, mycobacterium bovis, mycobacterium leprae or mycobacterium ulcerans.

It is particularly preferred, that the compounds according to the present invention are used in the treatment of tuberculosis.

Another embodiment of the present inventions relates to a pharmaceutical composition containing the compound according to the present invention for use in the prophylaxis and treatment of infectious disease including mycobycterial infection, like tuberculosis. The skilled person is well aware of suitable diluents, excipients, or carriers.

The pharmaceutical composition may be administered with a physiologically acceptable carrier to a patient, as described herein. In a specific embodiment, the term "pharmaceutically acceptable" means approved by a regulatory

agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, patches and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds according ot the present invention, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous or oral administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in a unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

"Therapeutically- or pharmaceutically-effective amount" as applied to the compositions of the instant invention refers to the amount of composition sufficient to induce a desired biological result. That result can be alleviation of the signs, symptoms, or causes of a disease, or any other desired alteration of a biological system. In the present invention, the result will typically involve a decrease in the load of the microorganism, e.g. of the Mycobacteria, as well as a decrease of the inflammation due to the infectious disease.

In vitro assays may optionally be employed to help identifying optimal dosage ranges. The precise dose to be employed in the formulation will also depend on the route of administration, and the seriousness of the disease or disorder, and should be decided according to the judgement of the practitioner and each patient's circumstances. Effective doses may be extrapolated from dose-response curves derived from in vitro or animal model test systems. Preferably, the pharmaceutical composition is administered directly or in combination with an adjuvant. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

In the context of the present invention the term "subject" means an individual in need of a therapy that can be alleviated or cured by administering the compounds according to the present invention to the individual. Preferably, the subject is a vertebrate, even more preferred a mammal, particularly preferred a human.

The term "administered" means administration of a therapeutically effective dose of the aforementioned pharmaceutical composition comprising the compounds according to the present invention.

The methods are applicable to both human therapy and veterinary applications. The compounds described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt%. The agents may be administered alone or in combination with other treatments.

The administration of the pharmaceutical composition can be done in a variety of ways as discussed above, including,

but not limited to, orally, subcutaneously, intravenously, intra-arterial, intranodal, intramedullary, intrathecal, intraventricular, intranasally, intrabronchial, transdermally, intrarectally, intraperitoneally, intramuscularly, intrapulmonary, vaginally, rectally, or intraocularly. In some instances, for example, in the treatment of wounds and inflammation, the pharmaceutically effective agent may be directly applied as a solution, a tablet or spray, in particular, in form of a tablet. The attending physician and clinical factors will determine the dosage regimen. A typical dose can be, for example, in the range of 0.001 to 5000 μg, like 0.1 to 1000 μg; however, doses below or above this exemplary range are envisioned, especially considering the aforementioned factors.

In one embodiment of the present invention, it is preferred that the salt according to formula II is a pharmaceutical acceptable salt. Moreover, the compounds according to the present invention may be present in form of its hydrates or solvates. The skilled person is well aware of suitable formulations.

Brief description of the drawings:

Fig (1): Intracellular activity of LX079 against M. bovis BCG in an infected macrophage model.
Fig. (2): Mean plasma concentration-time profile of LX079 in mice plasma following intravenous administration at 12.5 mg/kg. The straight line represents the in vitro MIC of LX079 against M. tuberculosis H37Rv
Fig. (3): Mean plasma concentration-time profiles of LX079 in mice plasma following oral administration at 12.5 and 25 mg/kg. The straight line represents the in vitro MIC of LX079 against M. tuberculosis H37Rv.
Fig. (4): In vitro metabolism by mouse liver microsomes of LX079: The substance was incubated with pooled mice hepatic microsomes in the presence of NADPH as a cofactor. The reaction was quenched at different time points. The percent of the parent substance was calculated in comparison to the concentration of the substance at time point 0.
Fig 5: Bacterial load in lung and spleen, overall results
Fig.6: Bacterial load in lung and spleen, treatment with Lx059
Fig.7: Bacterial load in lung and spleen, treatment with Lx069
Fig.8: Bacterial load in lung and spleen, treatment with Lx079.
Fig.9a and b: Examples of compounds useful according to the present invention

Materials & methods:

<u>Synthesis of compounds:</u>

The compounds of the present invention are prepared from known starting materials via various procedures following general methods described by us (Zapolskii et al 2007; Zapolskii et al 2010; Zapolskii et al 2012). For eample, 2-nitropentachloro-1,3-butadiene is easily accessible from trichloroethylene by radical dimerization, subsequent dehydrochlorination and, finally, nitration. The 1,1-bisbenzotriazole derivative is prepared by reaction of 2-nitropentachloro-1,3-butadiene with four equivalents of benzotriazole. 1-Substituted 4-nitro-5-dichloromethylpyrazoles (I) are formed by SNVin-reactions of benzotriazole derivative with various aminoalcohols and (het)arylhydrazines according to the known procedures (Scheme 1). Salts (II) are obtained by treatment pyrazoles (I) with corresponding acids.

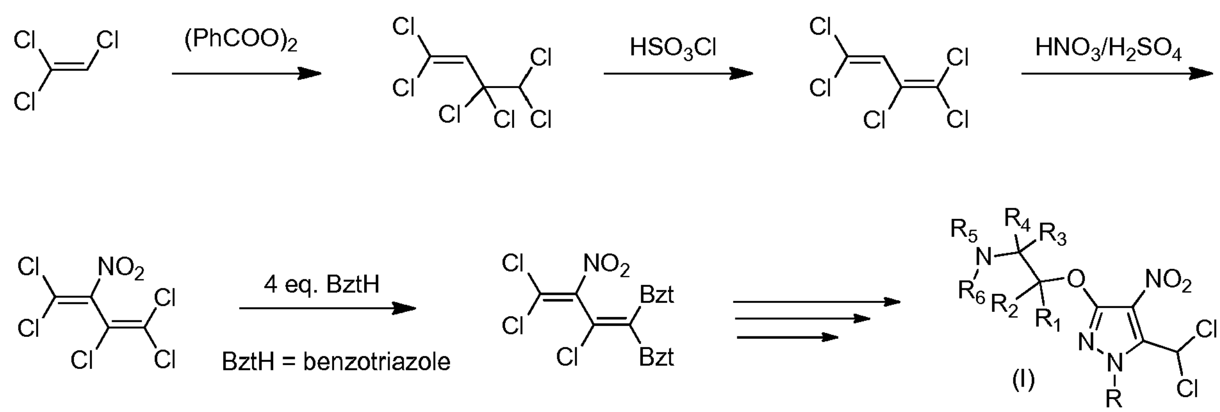


Scheme I

Melting points were determined with a Büchi apparatus 520 and are uncorrected. Thin layer chromatography (tlc) was

performed on Merck TLC-plates (aluminum based) silica gel 60 F 254. FTIR spectra were obtained with a Bruker Vector 22 FT-IR in the range of 400 to 4000 $cm^{-1}$ (2.5 % pellets in KBr). Mass spectra were obtained on a Hewlett Packard MS 5989B spectrometer, in direct mode with electron impact (70 eV). All peak values of molecular ions as well as fragments refer to the isotope $^{35}Cl$. $^1H$ NMR (600 MHz), $^{13}C$ NMR (150 MHz): Avance III 600 MHz FT-NMR spectrometer (Bruker, Rheinstetten, Germany). $^1H$ and $^{13}C$ NMR spectra were referenced to the residual solvent peak: $CDCl_3$, $\delta$= 7.26 ($^1H$), $\delta$= 77.0 ($^{13}C$) ; DMSO-$d_6$, $\delta$= 2.50 ($^1H$), $\delta$= 39.7 ($^{13}C$). Peak assignments were accomplished by results of HSQC- and HMBC-NMR experiments.

Minimal inhibitory concentration (MIC): The minimal inhibitory concentrations (MICs) of the substances were determined using resazurin microtiter assay (REMA) as described by Palomino (Palomino et al., 2002) in 96 well microplate. Mycobacterial strains were obtained from Lionex GmbH, Braunschweig, Germany. Resazurin sodium salt powders (Sigma) were prepared at concentration 0.01% in distilled water and filter sterilized. It was stored at 4°C and used within 1 week. The assay was performed in 7H9 medium (BD) supplemented with 10% ADC enrichment medium (Fluka) and 0.02% Tween-80 (Sigma). Two-fold serial dilutions of the tested compounds were prepared in 96 well plates in 7H9 medium with volume 100$\mu$l. When the bacteria reached early exponential phase ($OD_{550}$ =0.3), it was sonicated in ice bath for 30 sec and then diluted 1:20, and 100 $\mu$l was used as an inoculum. Growth controls containing no antibiotic and sterility controls without inoculation were also included. The plates were covered, sealed, and incubated at 37°C in the normal atmosphere. After 7 days, 30 $\mu$l of resazurin solution was added to each well and the plates were further incubated overnight at 37°C. The change of color was assessed; a change from blue to pink indicated reduction of resazurin and thus bacterial growth. The MIC is defined as the lowest substance concentration that prevented this color change.

**Assessment of in vitro cellular toxicity profile:**

Cell lines and media: Cells were grown at 37°C and 5% $CO_2$ in the following media supplemented with 10 % FBS(Gibco):

L-929 cells (mouse connective tissue fibroblast: Dulbecco's modified Eagle's medium (DMEM), Lonza
PtK2 cells (rat kangaroo kidney epithelial cells): Minimum Essential Medium (MEM) medium, supplemented with 1 % non-essential amino acids and 1 % glutamax, Invitrogen.

In vitro toxicity testing :

The effects of the tested compounds on cell proliferation and the acute toxic effects were assayed in vitro on L929 cell line. The metabolic activity was measured by means of (MTT, (3-(4,5-Dimethylthiazol-2-yl)-2,5-diphenyltetrazolium bromide) cell survival colorimetric assay.

Effect on proliferation: 60 $\mu$l of serial dilutions of the test compounds were added to 120 $\mu$l aliquots of a cell suspension (50,000 cells/ml) in 96-well microplates then incubated 5 days at 37°C and 5% $CO_2$. 20 $\mu$l MTT dye were added to a final concentration of 0.5 mg/ml. After 2 h the precipitate of formazan crystals was centrifuged, and the supernatant discarded. After washing with PBS, the precipitate was dissolved in 100 $\mu$l of acidified propanol. The plates were stirred for 20 minutes and the optical density of the resulting color was measured at 590 nm.

Acute toxic effect: It was investigated by plating 120 $\mu$l aliquots of a cell suspension (100,000-200,000 cells/ml) in 96-well microplates. The cells were incubated at 37°C and 5% $CO_2$ and allowed to grow for two days to stop the cell propagation. Then 60 $\mu$l of serial dilutions of the test compounds were added and incubated for further 24h. Then the MTT assay was performed as described before.

Results were expressed as the percentage of cellular viability in comparison to cells that were treated only with the vehicle. The $IC_{50}$ values were determined as the concentrations at which cells developed 50% of the absorbance of untreated control cells as estimated from the dose-response curves of two parallel experiments.

Immunofluorescence staining: The effects of substances on the phenotypical changes of cell shape and adhesion were investigatedin the potoroo (PtK2) cells after staining of the nucleus and the endoplasmic reticulum (ER). PtK2 cells were grown on cover slips in 4-well plates. semi-confluent cells were treated with test compounds and the plates were incubated over the night. The cells were fixed with cold methanol /acetone (1:1) for 10 min. Then the cells were washed with PBS and the primary antibody (anti GRP-94, thermoscientific) was added and incubated for 45 min and washed with PBS. Secondary antibody (Alexaflour goat anti-rat 488, Invitrogen) was then added to the cells and incubated for additional 45 min. Cells were washed with PBS and Cover slips were mounted in anti-fade mounting medium with DAPI (Invitrogen). Images were taken with a CCD camera attached to a fluorescence microscope.

Intracellular effectiveness:

To test the intracellular effectiveness of the test substance, BCG infected macrophage model was used and

treated with different sub-toxic concentrations. RIF was investigated in parallel as a control. For the infection experiment BCG were grown in7H9 medium (BD) supplemented with 10% ADC enrichment medium (Fluka), 0.02% Tween-80 (Sigma Aldrich) until it reached $OD_{600}$ =0.5 corresponding to $6*10^8$ CFU/ml. BCG then washed with PBS and resuspended in DMEM without FBS.

J774 a-1 macrophages growing in DMEM with 10% FBS were harvested, plated at a concentration of 5*105 cells per well in 24 well tissue plates, and incubated overnight at 37°C and 5% $CO_2$. The cells were overlaid with 1 ml of BCG suspension ($5*10^6$ /ml) adjusted to a multiplicity of infection 10. Cells were incubated 4 hours to allow phagocytosis to occur, and then they washed 4 times with PBS to eliminate the extracellular bacteria. Infected cells were overlaid again with culture medium containing the tested substances. Control wells were included with medium, uninfected macrophage, untreated infected macrophage, and infected macrophage that was treated with RIF. After 72 hours, the media from each well was transferred into corresponding 15 ml falcon tube containing 100μl 0.25% SDS. The cells were washed 4 times with 1 ml 0,025% SDS and added to the corresponding tube. BCG was pelleted by centrifugation at 2000g for 45 min then left to settle for 10 minutes. The supernatant was discarded and the pellet was resuspended in 1 ml 7H9 medium. Serial dilutions were plated onto 7H11 agar (BD) supplemented with 10 % OADC enrichment medium (Fluka). CFU were counted 2-4 weeks after incubation at 37°C and normal atmosphere.

*In vivo studies:*

All animal procedures were approved and supervised by the Animal Care Committee of the Germans TriasiPujol University Hospital and by the Department of Environment of the Catalan Government (approval number 4095). Mice were weighed and checked according to the protocols and observation data sheets that were requested. Animals were followed for weight loss, apparent good health (bristled hair and wounded skin) and behaviour (signs of aggressiveness or isolation).

Animals: Young adult (6-8 weeks old) female C57/BL6 mice, 20 mice for acute oral toxicity testing and 81 mice for PK experiment were used. The animals were nulliparous and non-pregnant weighing 20 ±1.5 g each. They were maintained in their cages provided with a standard laboratory rodent diet and unlimited supply of drinking water and were allowed to acclimatize to the laboratory conditions for seven days before starting the experiments. The temperature in the experimental animal room was kept at 22°C ± 3°C and the relative humidity was 50 ± 20 %. Lighting was artificial with the sequence 12 hours light and 12 hours dark.

Substance formulation for in vivo model: Doses were prepared in 5% DMSO in sterile water for oral dosing through oral gavage at a dose volume of 0.3 ml, and in 5% DMSO in PBS, pH 7.4 for IV bolus injection via tail vein at a dose volume 0.1 ml.

Testing of acute oral toxicity in mice: The oral acute toxicity study was carried out using 20 mice that were randomly distributed into one control group and 3 treated groups, containing five animals per group. The control group received the vehicle while each treated group received the substance at doses 62.5, 125 or 250 mg/kg as a single oral dose. The animals were observed continuously for the first 4 h and then daily for the following 14 days, to observe any death or changes in general behavior and other physiological activities. Animals were weighted at the time of dosing then after 1 week and at the end of the experiment. Animals were observed for morbidity conditions, pain, skin, fur, respiratory distress, allergic reactions, eyes, salivation, diarrhea, behavior (hypo-hyper reactivity), tremors, convulsions, ataxia, lethargy or coma. At the end of the experiment mice were sacrificed and the internal organs were observed for the presence of any gross necropsy, the histopathology was done for the liver, spleen, lungs and kidneys in the control and highest dose groups. The cumulative mortality rate was calculated using Reed-Muench method.

In vivo pharmacokinetics:

For studying the PK profile of the LX079, 81 mice were used each received single dose either oral or IV. The animals were divided into one IV group (27 mice), 2 oral groups (24 mice each), control IV group (3 mice) and control oral group (3 mice). The IV group and one oral group were dosed with 12.5 mg/kg and the other oral group was dosed with 25 mg/kg, while the control groups received the vehicle. All blood samples were collected at 15 and 30 min and 1, 2, 4, 6, 8, and 24 h after oral dosing, and at 5, 15, and 30 min and 1, 2, 4, 6, 8, and 24 h following intravenous administration. The blood was collected from the control groups at 24 h. Mice were euthanized with isoflurane and a 0.3 mL aliquot of blood was collected via terminal bleed from three mice at each time point. Blood samples were collected in heparin as anticoagulant and plasma was harvested by centrifugation at 10,000 rpm for 2 minutes. Plasma samples were kept at -80 °C until analysis.

Hepatic microsomal stability and 1st pass effect: Pooled mouse microsomes (protein concentration, 20 mg/mL) were purchased from XenoTech (Lenexa, KS). NADPH was purchased from Sigma. Compound stock solution was prepared in DMSO. The final concentration of DMSO in the reaction was kept below 0.2% (v/v). The stability of LX079 in microsomes was determined after its incubation at 1 μM with microsomes (0.5 mg/mL) in 50 mM potassium phosphate buffer (pH

7.4) at 37 °C. The total reaction volume was 100 μL and the reaction mixture was first warmed at 37 °C for 5 min before adding NADPH (1.0 mM). Reactions were quenched at 0, 5, 10, 15, 20, 30 min by adding acetonitrile (300 μL). Control reactions contained no NADPH were included. The samples were filtered using miniuniprep filter PTFE 0.2 μM (Whatman) and then analyzed with UHPLC/MS. The hepatic intrinsic clearance was estimated by scaling up the in vitro $t_{1/2}$.
Equilibrium dialysis for plasma protein binding: The rapid equilibrium dialysis device (RED, Pierce) with cutoff-mass of dialysis membrane 8,000 daltons was used. Pooled mice plasma was prepared by $K_3EDTA$ and by centrifugation for 10,000 for 2 min. The substance was prepared at a concentration of 1μM and 2 μM in plasma and the Conc. of the organic solvent was kept below 1%. 300μl plasma was dialyzed against 500 μl PBS at 37°C for 4 hours. The dialysis device was sealed properly and vortexed 500 rpm on a plate vibrator for achieving equilibrium. Nonspecific binding to the membrane was excluded by running control experiments with buffer in the both chambers. At the end of dialysis 100 μl of plasma and buffer was collected and precipitated with 300p1 acetonitrile. The samples were filtered using miniuniprep filter PTFE 0.2 μM (Whatman) and then analyzed with UHPLC/MS.
For determination of the degree of binding to hepatic microsomal enzymes, 2 μM of the substance was prepared in pooled hepatic microsomes diluted to 1 mg/ml in PBS and then 100μl of pooled hepatic microsomes was dialyzed against 300p1 PBS.

UHPLC/MS analysis:

For quantification, calibration standards for LX079 were prepared in normal mice plasma by two-fold serial dilutions. After protein precipitation and filtration as described above, the parent substance was quantified in the samples using LC/MS in the cooperation with Knauer GmbH, Germany. The LC-MS analysis was performed using PLATINblue UHPLC-MSQ system (Knauer, Germany). Reversed phase liquid chromatography separations during LC-MS were carried out by using BlueShell 80-2.6 C18, 100 x 2 mm ID column connected to the UHPLC system. The LC separation was performed using a binary gradient consisted of a mixture of 0.1% formic acid in water (solvent A) and 0.1% formic acid in acetonitrile (solvent B). The gradient (at flow rate of 0.6 ml/min) was programmed as follows: 0 - 2 min (40 % B), 2 - 2.2 min (40 - 100 % B) and 2.2 - 3 min (100 % B). The column temperature was 35°C and the injection volume was 5 μl. A full scan in the range of 20 - 1000m/z was performed to find the mass of the target compound. The highest signal was reached for m/z = 490 and itwas chosen for quantification. The electrospray ionization (ESI) was conducted in the positive ionization mode with needle voltage 4 KV and cone voltage 75 V. The capillary temperature was 500°C.

Pharmacokinetic analysis:

The area under the 24 hours plasma concentration-time curve (AUC) was calculated using the linear trapezoidal rule. Following IV bolus administration, half-life ($t_{1/2}$) and plasma clearance (CL) were calculated using the following equations where K is the elimination rate constant of the one phase decay (Shahab et al, 2010).

$$t_{1/2} = 0.693 / K$$

$$CL = Dose / AUC$$

Following oral dosing, absolute bioavailability (F) was calculated from the following equation where ($AUC_{PO}$) and ($AUC_{IV}$) are the areas under the 24 hours plasma concentration-time curves following oral and IV administration respectively (Shahab et al, 2010).

$$F = (AUC_{PO} / AUC_{IV}) * (dose_{IV} / dose_{PO})$$

The in vitro stability with the hepatic microsomal enzymes was calculated as follows:

$$Stability\ (\%) = 100 \times Conc.\ at\ 30\ min / Conc.\ at\ 0\ min$$

The In vitro $t_{1/2}$ was calculated from the following equation, where K is the elimination rate constant obtained from the slope of disappearance kinetics (in the form of relative remaining concentration of the parent substance versus time).

$$t_{1/2} = 0.693 / K$$

The In vitro clearance ($CL_{int, inc}$) was calculated from the equation:

$$CL_{int, inc} = k * (V/M) / F_{ub}$$

Where V is the volume of incubation, M is the amount of microsomes in the incubation and $F_{ub}$ is the unbound fraction. $F_{ub}$ was calculated from the results of the equilibrium dialysis as follows:

$$F_{ub} = \text{Conc. in buffer side / Conc. in side of microsomes}$$

The hepatic intrinsic clearance $CL_{int}$ from microsomal incubation in turn was calculated as:

$$CL_{int} = CL_{int,inc} * \text{MPPGL} * \text{weight of liver}$$

Where MPPGL is the scaling factor for microsomal protein per gram of liver (Davies & Morris, 1993) (Nassar et al., 2009) (Houston, 1994) (Naritomi et al., 2001) (www.admescope.com) (www.cyprotex.com).

**Results:**

<u>Synthesis of compounds:</u>

TABLE 1

| The following samples of 4-nitro-5-dichloromethylpyrazoles (I) and (II) have been prepared: | | | | |
|---|---|---|---|---|
| Structure | Abbreviation | Chemical name | Yield , % | Mp, °C |
| 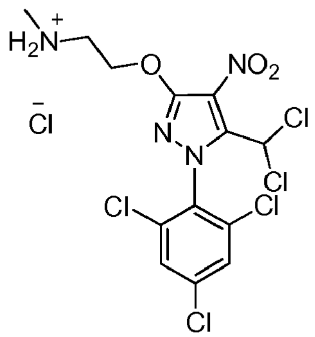 | Vz0825 or Lx079 | 2-((5-(Dichloromethyl)-4-nitro-1-(2,4,6-trichlorophenyl)-1H-pyrazol-3-yl)oxy)-N-methylethanaminium chloride | 55 | 204-205 |
| 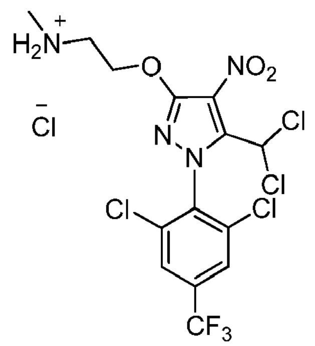 | vz2090 or Lx088 | 2-((1-(2,6-Dichloro-4-(trifluoromethyl)phenyl)-5-(dichloromethyl)-4-nitro-1H-pyrazol-3-yl)oxy)-N-methylethanaminium chloride | 27 | 205-207 |

(continued)

| The following samples of 4-nitro-5-dichloromethylpyrazoles (I) and (II) have been prepared: | | | | |
|---|---|---|---|---|
| Structure | Abbreviation | Chemical name | Yield , % | Mp, °C |
| 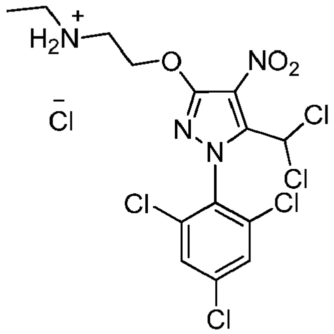 | vz2106 or Lx089 | 2-((5-(Dichloromethyl)-4-nitro-1-(2,4,6-trichlorophenyl)-1H-pyrazol-3-yl)oxy)-N-ethylethanaminium chloride | 29 | 193-195 |
| 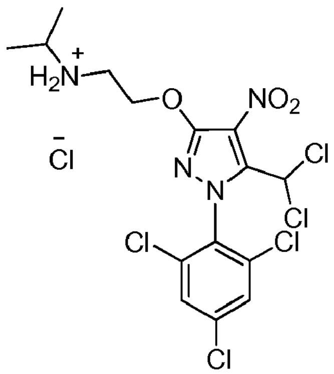 | vz2109 | N-(2-((5-(Dichloromethyl)-4-nitro-1-(2,4,6-trichlorophenyl)-1H-pyrazol-3-yl)oxy)ethyl)propan-2-aminium chloride | 30 | 191-193 |
| 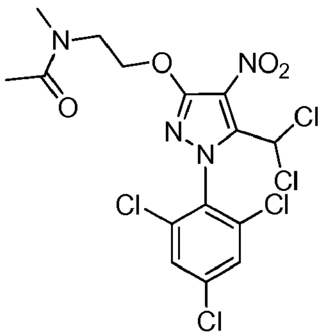 | vz2082 | N-(2-((5-(Dichloromethyl)-4-nitro-1-(2,4,6-trichlorophenyl)-1H-pyrazol-3-yl)oxy)ethyl)-N-methylacetamide | 86 | Oil |

Synthesis of vz2088

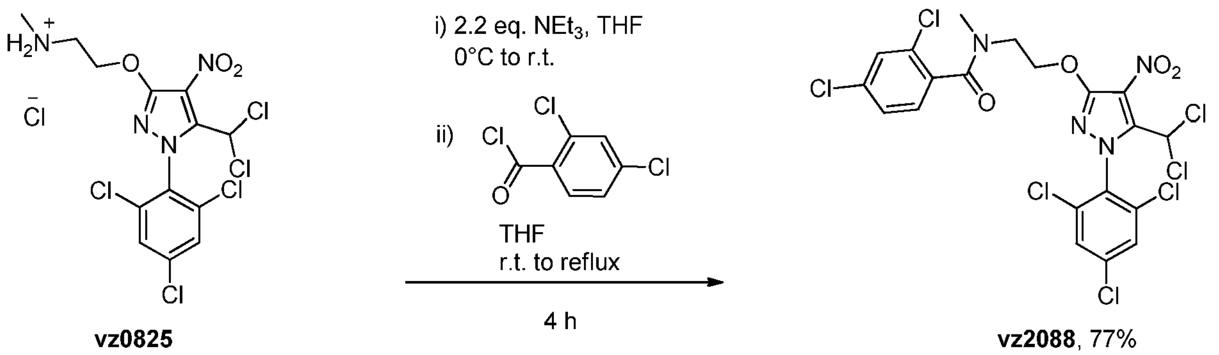


**vz0825** **vz2088**, 77%

To a suspension of 60.0 mg (0.124 mmol) of vz0825 in 5 mL dry THF was added a solution of 28.0 mg (2.2 eq, 0.273 mmol) triethylamin in dry THF (1 mL) at 0°C within 5 min with stirring. The resulting reaction mixture then was stirred for 1 h at room temperature. Subsequently, 29.0 mg (1.1 eq., 0.136 mmol) of 2,4-dichlorobenzoyl chloride in dry THF (1 mL) was added and resulting reaction mixture was refluxing for 3 h. After evaporation of the solvent in vacuo, 10 mL of water and 1 mL of conc. HCl were added with stirring. The reaction mixture was extracted with chloroform (3 x 10 mL). The organic phase was washed with water (1 x 20 mL) and dried with calcium chloride. After evaporation of the solvent in vacuo and purification of product with column chromatography (petrolether/ethyl acetate 5:1) pyrazole vz2088 was obtained as yellow powder; yield: 59.3 mg (0.095 mmol, 77 %); m.p. 68-69 °C. Pyrazole vz2088 is formed as mixture of two atropisomers in ratio 68:32.

Physical-chemical data of vz2106

(2-((5-(Dichloromethyl)-4-nitro-1-(2,4,6-trichlorophenyl)-1H-pyrazol-3-yl)oxy)-N-ethylethanaminium chloride)

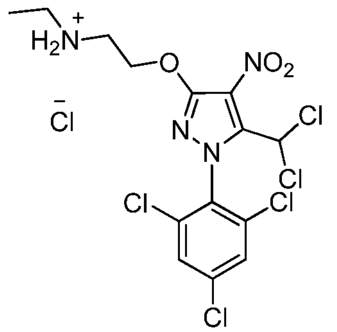

Yield 302 mg (0.605 mmol, 29%). M.p. 193-195°C. IR (KBr): v = 2980, 2705, 1578 ($NO_2$), 1511, 1483, 1354 ($NO_2$), 1185, 1013, 929, 861, 826, 738, 581, 564, 452 $cm^{-1}$. - $^1H$ NMR ($CDCl_3$): δ= 9.89 (broad s, 2 H, $NH_2$), 7.52 (s, 2 H, Ar), 7.37 (s, 1 H, $CHCl_2$), 4.83 (t, 2 H, J = 5.0 Hz, $OCH_2$), 3.53-3.45 (m, 2 H, $NCH_2$), 3.36-3.26 (m, 2 H, $NCH_2$), 1.48 (t, 3 H, J = 7.2 Hz, $CH_3$). - $^{13}C$ NMR ($CDCl_3$): δ= 155.6 (C=N), 140.0 ($CCHCl_2$), 138.2 ($C_{Ar}Cl$), 136.3 (2 $C_{Ar}Cl$), 132.3 ($C_{Ar}N$), 129.0 (2 $CH_{Ar}$), 118.5 ($CNO_2$), 65.8 ($OCH_2$), 57.0 ($CHCl_2$), 44.7 ($NCH_2$), 43.4 ($NCH_2$), 11.4 ($CH_3$). - MS (only cation): m/z (%) = 461 (6) [$M^+$], 447 (10) $[M-CH_3]^+$, 373 (100) [M-EtNH$_2$
$C_2H_4O$+H]$^+$, 179 (2,4,6-trichlorophenyl, 45).

Physical-chemical data of vz0825

2-((5-(Dichloromethyl)-4-nitro-1-(2,4,6-trichlorophenyl)-1H-pyrazol-3-yl)oxy)-N-methylethanaminium chloride

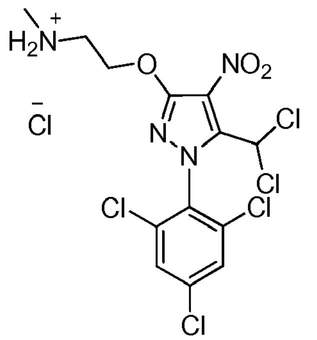

Yield 55%. M.p. 204-205°C. IR (KBr): v = 3091, 2952, 2723, 2471, 1581 ($NO_2$), 1516, 1486, 1366 ($NO_2$), 1281, 1194, 1103, 1008, 918, 860, 833, 744, 574, 565, 452 $cm^{-1}$. - $^1H$ NMR (DMSO-$d_6$): δ= 9.33 (broad s, 2 H, $NH_2$), 8.10 (s, 2 H, Ar), 7.58 (s, 1 H, $CHCl_2$), 4.63 (t, 2 H, J = 5.0 Hz, $OCH_2$), 3.38-3.31 (m, 2 H, $NCH_2$), 2.62 (broad s, 3 H, $CH_3$). $^{13}C$ NMR (DMSO-$d_6$): δ= 156.3 (C=N), 140.7 ($CCHCl_2$), 138.0 ($C_{Ar}Cl$), 135.2 (2 $C_{Ar}Cl$), 131.2 ($C_{Ar}N$), 129.7 (2 $CH_{Ar}$), 119.3

($CNO_2$), 66.2 ($OCH_2$), 58.5 ($CHCl_2$), 46.6 ($NCH_2$), 33.0 ($CH_3$). - MS (only cation): m/z (%) = 447 (8) [$M^+$], 400 (5) [M-$HNO_2$]$^+$, 373 (100) [M-$MeNH_2C_2H_4O$+H]$^+$, 267 (9) [M-ArH]$^+$.

Physical-chemical data of vz2109

N-(2-((5-(dichloromethyl)-4-nitro-1-(2,4,6-trichlorophenyl)-1H-pyrazol-3-yl)oxy)ethyl)propan-2-aminium chloride

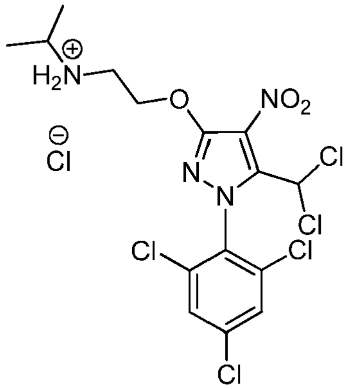

Yield 30%. M.p. 191-193°C. IR (KBr): v = 2977, 2690, 2455, 1575 ($NO_2$), 1511, 1484, 1360 ($NO_2$), 1183, 1103, 1019, 857, 830, 821, 748, 565, 430 $cm^{-1}$ . - $^1H$ NMR ($CDCl_3$): δ= 9.73 (broad s, 2 H, $NH_2$), 7.51 (s, 2 H, Ar), 7.37 (s, 1 H, $CHCl_2$), 4.82 (t, 2 H, J = 5.1 Hz, $OCH_2$), 3.73-3.62 (m, 1 H, CH), 3.52-3.45 (m, 2 H, $NCH_2$), 1.47 (d, 6 H, J = 6.5 Hz, $CH_3$). - $^{13}C$ NMR ($CDCl_3$): δ= 155.6 (C=N), 140.0 ($CCHCl_2$), 138.2 ($C_{Ar}N$), 136.2 (2 $C_{Ar}Cl$), 132.3 ($C_{Ar}Cl$), 129.0 (2 $CH_{Ar}$), 118.4 ($CNO_2$), 65.9 ($OCH_2$), 57.0 ($CHCl_2$), 50.7 (CH), 42.2 ($NCH_2$), 19.1 ($2CH_3$). - MS (only cation): m/z (%) = 475 (8) [$M^+$], 461 (100), 392 (10) [M-$CHCl_2$]$^+$, 373 (18) [M-$Me_2CHNH_2C_2H_4O$+H]$^+$, 179 (57) [Ar]$^+$.

Physical-chemical data of vz2090

2-((1-(2,6-dichloro-4-(trifluoromethyl)phenyl)-5-(dichloromethyl)-4-nitro-1H-pyrazol-3-yl)oxy)-N-methylethanaminium chloride

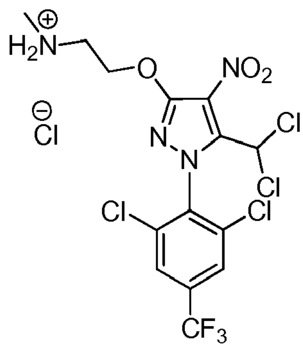

Yield 27%. M.p. 205-207°C. IR (KBr): v = 2700, 1575 ($NO_2$), 1514, 1489, 1363 ($NO_2$), 1325, 1302, 1183, 1129, 1010, 932, 891, 819, 755, 713, 575, 555 $cm^{-1}$. - $^1H$ NMR (DMSO-$d_6$): δ= 9.58 (broad s, 2 H, $NH_2$), 8.32 (s, 2 H, Ar), 7.59 (s, 1 H, $CHCl_2$), 4.64 (t, 2 H, J = 5.1 Hz, $OCH_2$), 3.37-3.31 (m, 2 H, $NCH_2$), 2.61 (broad s, 3 H, $CH_3$). - $^{13}C$ NMR (DMSO-$d_6$): δ= 156.4 (C=N), 140.6 ($CCHCl_2$), 135.7 (2 $C_{Ar}Cl$), 135.5 ($C_{Ar}N$), 134.0 ($CCF_3$, J = 33.6 Hz), 127.1 (2 $CH_{Ar}$, J = 3.8 Hz), 122.3 ($CF_3$, J = 273.5 Hz), 119.4 ($CNO_2$), 66.3 ($OCH_2$), 58.4 ($CHCl_2$), 46.5 ($NCH_2$), 32.9 ($CH_3$). - MS (only cation): m/z (%) = 481 (2) [$M^+$], 407 (50) [M-$MeNH_2C_2H_4O$+H]$^+$, 213 (28) [Ar]$^+$.

Primary screening: Evaluating the in vitroantituberculous activity:

A library of about 480 compounds was screened against M. tuberculosis H37Rv using REMA. Out of the 480 compounds, 4 compounds had low or submicromolar MIC (Table-2).

Table (2) Minimal inhibitory concentration (MIC), $IC_{50}$ and selectivity index (SI) of the selected compounds:

| | (MIC) against M. tuberculosis H37Rv(μg/ml) | $IC_{50}$ (μg/ml) in MTT | | SI ($IC_{50}$/MIC) with L929 cell line | |
|---|---|---|---|---|---|
| | | 5 days | 1 day | 5 days | 1 day |
| vz1172 | 0.32 | 0.6 | 6.8 | 1.88 | 21.3 |
| vz1070 | 0.15 | 2 | 9.8 | 13.3 | 65.3 |
| vz0825 | 0.18 | 2.2 | 5.5 | 12.2 | 30.6 |
| vz0536 | 0.19 | 0.1 | 1.29 | 0.53 | 6.8 |

Secondary screening: Evaluating the in vitro toxicity profile against mammalian cells:

Subsequently, the in vitro cytotoxicity and SI of the 4 compounds were assessed using the MTT assay with L929 cell line after 1 and 5 days. The MIC, $IC_{50}$ and the selectivity index (SI) of the 4 compounds are listed in (Table-2). The SI was calculated by dividing the $IC_{50}$over the MIC. In between the 4 compounds the, LX077 and LX079 had the most promising results. When the SI was calculated by the $IC_{50}$ obtained after 5 days incubation with the substances, their SI were exceeding 10. And their SI when calculated from $IC_{50}$ from the acute toxic effect experiment, their SI were 30.6 and 65.3 respectively.

Intracellular effectiveness:

As presented in (Fig. 1) Lx079 exhibited significant intracellular activity against M. bovis BCG. At concentration 2 μg/ml it was able to decrease the log CFU by around 2 in comparison to the untreated controls showing that ven though serum levels decrease rapidly, Lx079 accumulates in macrophages to effective leves and controls the growth of the live mycobacteria.

Acute oral toxicity:

The acute oral toxicity of LX079 was evaluated in C57/BL6mice after single oral dose. The animals were observed for 14 days after dosing. The characteristic observed signs and the survival of animals are summarized in table (3). 100 % survival was recorded in the animals that received 62.5 mg/kg, However, the mice showed slight tremors after 4 hours of the treatment which recovered during the first 24 hours. The percentages of cumulative deaths between the animals received 125 and 250 mg/kg were 22 and 42.85 % respectively (table-4). The animals that received 125 and 250 mg/kg presented with respiratory distress and neurological signs like tremors and sleep. These toxic manifestations recovered slowly during the first 24 hours and then the surviving animals exhibited normal appearance and behavior. While the 3 animals that were decided to be humanely killed started to convulse. There was no significant change in the body weight between the treated and the control groups; however the animals that received 125 mg/kg showed weight loss after the first week which was increased again normally by the end of the experiment (table-5). Macroscopic examinations did not show any changes in the appearance of the organs of the treated animals compared with the control group. No pathological abnormalities were detected by microscopic examination of liver, spleen, lungs, and kidneys in all tested groups.

Table (3): Characteristic signs and survival of C57/BL6 mice at different doses of LX079.

| Dose (mg/kg) | Time post dosing | Characteristic signs |
|---|---|---|
| 62.5 | 30-240 min | Normal appearance and behavior as healthy mice |
| | 240 min | Appearance of slight tremors |
| | 240 min-24 hr. | Apparent recovery |
| | Following 14 days | Normal appearance and behavior as healthy mice |

(continued)

| Dose (mg/kg) | Time post dosing | Characteristic signs |
|---|---|---|
| 125 | 30-240 min | Three mice showed respiratory distress, tremors, lethargy and sleep. While the other 2 mice showed normal appearance and behavior. |
| | 240 min | One of healthy mice started to show bad skin conditions |
| | 240 min-24 hr. | 2 mice with tremors and respiratory distress start to convulse, and they were sacrificed to avoid more suffering. The others start to recover |
| | Following 14 days | The remaining surviving 3 mice showed normal appearance and behavior as healthy mice. |
| 250 | 30-180 min | 3 mice showed respiratory distress, tremors and sleep. |
| | 180-240 min | The other 2 animals started to show tremors and sleep |
| | 240 min | One mouse showed convulsion and it was humanely killed to avoid more suffering. |
| | 240 min-24 hr. | Recovery of the remaining 4 animals |
| | Following 14 days | The remaining surviving 4 mice showed normal appearance and behavior as healthy mice. |

Table (4): The acute oral toxicity of LX079 in C57/BL6 mice.

| Group | No. of Mice | Dose (mg/kg) | No. of dead mice | % Cumulative of dead mice* | Macroscopic examina-tion (liver, lungs, kidneys, spleen) |
|---|---|---|---|---|---|
| 1 | 5 | control | 0 | 0 | Normal |
| 2 | 5 | 62.5 | 0 | 0 | Normal |
| 3 | 5 | 125 | 2 | 22 | Normal |
| 4 | 5 | 250 | 1 | 42.85 | Normal |
| * using Reed-Muench method. | | | | | |

Table (5): Effect of administering different doses of LX079 on body weight of C57/BL6mice over a period of two weeks.

| Dose (mg/kg) | Day 0 | Day 7 | Day 14 |
|---|---|---|---|
| Control | 19.42 ± 0.53 | 19.62 ± 0.33 | 19.9 ± 0.7 |
| 62,5 | 19.62 ± 0.68 | 20 ± 0.8 | 20.3± 0.37 |
| 125 | 19.13 ± 0.49 | 18.87 ± 0.74 | 20.2 ± 0.85 |
| 250 | 18.88 ± 0.15 | 19.15 ± 0.31 | 19.65 ± 0.47 |

The results are presented as the mean ± standard deviation.

The in vivo pharmacokinetics (PK) of LX079 was evaluated inC57/BL6 mice after oral and IV administration. The plasma concentration-time profile after 12.5 mg/kg single IV dosing is shown in (Fig. 2) and the related PK parameters are listed in (table-6). The plasma concentration-time profiles of LX079 after 12.5 and 25 mg/kg single oral administration are shown in (Fig. 3). (Table -7) shows the oral bioavailability with each dose.

Table (6) Pharmacokinetic parameters of LX079 in mice plasma following intravenous administration at 12.5 mg/kg.

| | |
|---|---|
| C0 ($\mu$g/ml) | 0.66 ± 0.07 |
| AUC (h.$\mu$g.ml$^{-1}$) | 0.53 ± 0.03 |
| $t_{1/2}$ (h) | 0.43 ± 0.27 |
| K | 1.79 ± 0.44 |
| CL (l. h$^{-1}$ . kg$^{-1}$) | 23.64 ± 1.22 |

Values are represented as mean ± SD. n = 3 at each time point. AUC: Area under the 24 hour plasma concentration-time curve.$t_{1/2}$: Half-life. K: Elimination rate constant. CL: clearance.

Table (7):Pharmacokinetic parameters of LX079 in mice plasma following oral administration at 12.5 and 25 mg/kg.

| Dose (mg/kg) | AUC (h.μg.ml$^{-1}$) | F (%) |
|---|---|---|
| 12.5 | 0.15 ± 0.02 | 27.58 ± 2.46 |
| 25 | 0.35 ± 0.07 | 32.66 ± 7.72 |

Values are represented as mean ± SD. n = 3 at each time point. AUC: Area under the 24 hour plasma concentration-time curve. F: Bioavailability.

After iv administration of LX079 intravenously, it exhibited $t_{1/2}$ of 0.43 ± 0.27 and the peak plasma concentration was 0.66 ± 0.07. The time in which the plasma concentration was exceeding the in vitro MIC (T > MIC) was less than 1 hour However, the initial plasma concentration level indicates large volume of distribution. After oral administration at 12.5 and 25 mg/kg the oral bioavailability was estimated 27.58 ± 2.46 and 32.66 ± 7.72 respectively and the plasma levels did not reach the level of the in vitro MIC.

<u>In vitro pharmacokinetic studies:</u>

The stability of the compound with hepatic microsomes was investigated. In vitro incubation of LX079 with pooled mice hepatic microsomes resulted in in vitro $t_{1/2}$ of 5.94 ± 2.36 (Fig. 4). The in vitro clearance ($CL_{int, inc}$) was calculated with the consideration of the binding to microsomes. The hepatic intrinsic clearance ($CL_{int}$) was estimated by scaling up the in vitro $t_{1/2}$(Table-8).

Table (8): Metabolic stability parameters of LX079 in mice microsomes.

| | |
|---|---|
| $t_{1/2}$ (min) | 5.937 ± 2.36 |
| $CL_{int, inc}$ (ml/min/mg protein) | 2.82 ± 1.12 |
| $CL_{int}$(ml/min/SMW) | 221.81 ± 88.23 |
| Stability (%) | 23.14 ± 2.01 |
| SMW: Standard mouseweight. | |

<u>Effectivity in mice</u>

Lx079 as well as two other components, Lx059 which is 10% DMSO in PBS buffer and Lx069 which is a combination of ethambutol (0.8 mg/ml) and myxopyronine (0.2 mg/ml), were tested for drug effectivity in mice. Two controls were included in the study (sham, non treated; and treated with Isoniazide (INH, 25 mg/kg).

The animals were infected at time point 0 with M. tuberculosis through an airborne infection apparatus (Glas-col Inc., Terre Haute, IN, USA) for infection with a low dose of H37Rv Pasteur M. tuberculosis strain. Nebulization provided an approximate uptake of 20-50 bacilli by mice lungs.

The treatments and controls results were processed blindly. The experiment was run including 10 C57BL/6 mice per group. A volume of 0.2mL of each treatment was administered orally every day for 5 days, on week 2 post infection.

All animal procedures were approved and supervised by the Animal Care Committee of the Germans TriasiPujol University Hospital. Mice were weighed and checked every day following a protocol that monitored weight loss, apparent good health (bristled hair and wounded skin) and behaviour (signs of aggressiveness or isolation), in order to detect any suffering and/or any sign of toxicity.

Mice were euthanized on week 3 with isoflurane (inhalation excess), and lung and spleen samples removed to and mechanically disrupted in order to obtain tissue homogenates to be plated cultured in Becton Dickinson 7H11 Middlebrook agar (Bennex Ltd, Shannon, Ireland) and incubated at 37ºC. Viable bacteria (Colony Forming Units, CFUs) were counted four weeks after, data being recorded as the log of the number of CFUs recovered per organ. The results of the different groups were represented graphically, compared and evaluated for statistically significant differences (non parametric comparison between each candidate and each control, Mann Whitney test), with the differences being considered significant at $P \leq 0.05$ (Graphpad software).

<u>INCIDENCES</u>

A total of 7 mice instead of 10 were included in the Lx069-1 group, because of being short of product.

RESULTS

No toxicity signs were detected during the whole period the experiment last, as the candidates were well tolerated.

The effect on the bacillary load of each candidate results have been logarithmically represented as CFU/mL in the graphs attached. The lung and spleen results of each candidate and controls evaluated have been represented together (as box and whiskers, 5-95 percentiles plotted) and also per candidate with sham (results of each mouse and median and standard deviation plotted). Statistically significant differences ($p<0.05$) are marked with * if existing between the evaluated group and sham. All candidates demonstrated to be different to the sham control in a statistically significant way, either in lungs, spleen, or both.

In figure 5 the overall results are shown. In figure 6, the results for Lx059 are shown. Lx059 decreased the bacillary load in spleen when compared to sham, in a statistically significant way (p=0.00117).

The effect of Lx069-1 on the bacillary load was statistically significant both in lungs and spleen, when compared to sham, achieving a decrease of approximately half a log (p=0.0007, in lungs; p=0.0059, in spleen), see figure 7.

Up to 6 doses of the same candidate (Lx079) were evaluated, see figure 8. All of them were found to decrease the bacillary load in both in lungs and spleen, when compared to sham.

The p obtained in the statistical analysis are shown in the table below:

| Candidate | p (Lungs) | p (Spleen) |
|---|---|---|
| Lx079-1 | 0.0032 | 0.0009 |
| Lx079-2 | 0.0002 | 0.0343 |
| Lx079-3 | 0.0010 | 0.0002 |
| Lx079-4 | 0.0004 | 0.0117 |
| Lx079-5 | 0.0002 | 0.0164 |
| Lx079-6 | 0.0002 | 0.0031 |

MTT assay results of LX079 analogues:

The MTT assay was carried out after incubating different concentrations of the substances with J774 a.1 macrophage cell line for 5 days. The $IC_{50}$ was calculated from the dose response curve as the dose was inhibited the 50% of cell viability. The ratio between $IC_{50}$ and MIC against M. bovis BCG was used to calculate the selectivity index (SI) of each compound as an estimate of the therapeutic window as shown in the following table.

| | MIC ($\mu$g/L) | $IC_{50}$ ($\mu$g/L) | SI (IC50/MIC) |
|---|---|---|---|
| LX079 | 600 | 2000 | 3.33 |
| Lx080 vz0536 | 391 | 280 | 0.72 |
| Lx088 | 781 | 6000 | 7.68 |
| Lx089 | 781 | 2000 | 2.56 |
| Compound Lx080, see figure 9. | | | |

Conclusion:

- All compounds exhibited SI less than 10 (narrow therapeutic window).
- LX088 exhibited the highest SI of 7.68 which is greater than the SI of LX079.
- Investigating the morphological effect by immunohistochemistry following the treatment with LX088 is recommended.

Effect on cell morphology using ICC :

The effects of substances on the phenotypical changes of cell shape and adhesion were investigated by the technique of immunocytochemistry (ICC). The endoplasmic reticulum (ER) was investigated upon treatment of the potoroo cells (PtK2) cells with/without test compounds.

Procedure:

PtK2 cells were grown on cover slips in 4-well plates. semi-confluent cells were treated with test compounds and the plates were incubated over the night. The cells were fixed with cold methanol /acetone for 10 min. Then the cells were washed with PBS and the primary antibody (anti GRP-94, thermoscientific) was added and incubated for 45 min and washed with PBS. Secondary antibody (Alexaflour goat anti-rat 488, Invitrogen) was then added to the cells and incubated for additional 45 min. After washing with PBS, Cover slips were mounted in anti-fade mounting medium with DAPI. Images were taken with a CCD camera attached to a flourescence microscope.

Conclusion:

- LX079 at conc. 5μg/ml did not produce morphological abnormalities.
- LX079 at conc. 6.6 μg/ml induced massive ER stress.
- LX088 produced less ER stress at conc. 6.6 μg/ml in comparison to LX079 at the same conc.
- LX088 at conc. 13.3 μg/ml induced massive ER stress.

Discussion:

The whole-cell based approaches had contributed to identify all anti-TB drug candidates that are currently in clinical trials by predicting their ability to kill the pathogen in vitro.REMA was applied where ≈480 compounds were tested againstM.tb. Based on this screening, 5 molecules inhibited the microbial growth with micromolar concentrations were selected to evaluate their toxicity profile. Hits should be essentially and carefully evaluated for their cytotoxicity profiles on mammalian cells before taking them ahead into the following steps. Here, cytotoxicity of the 5 hits was estimated against L929 cell line by using MTT cell proliferation assay. It was observed that there was no significant effect on proliferation of L929 cell line at a concentration 10 times higher than MIC levels of 2 hits. After immunofluorescence staining, the LX079 was the only hit that showed no morphological changes of the treated cells at a concentration 25 times higher than the MIC. This data indicated that LX079 could be selected for further research. In antimicrobial in vitro models, MIC less than 2 μg/mL with SI more than 10 is satisfactory for further development (Sanchez&Kouznetsov, 2010). In addition, the compound exhibited intracellular activity when tested in an infected macrophage model.
To enable further studies, the acute toxicity of the new compound was evaluated in mice. And according to the OECD guidelines for testing toxicity of chemicals (425-OECD guideline for testing of chemicals), animals should be followed for 14 days after dosing to reveal any delayed toxicity. By calculating the cumulative percentage of death between the mice, the $LD_{50}$ was estimated to be more than 250 mg/kg. However; animals that were dosed with 62.5 mg/kg manifested with slight tremors and with higher doses animals showed respiratory distress with neurological manifestations as tremors, lethargy and sleep. All manifestations in the surviving animals disappeared during the first 24 hours and no delayed toxic signs were predicted during the whole period of the experiment. As well as no significant histopathological or body weight changes were detected in all tested groups.
The PK profiles for the LX079 were evaluated in mice following oral and IV administration.The LX079 exhibited in vivo PK pattern with short $t_{1/2}$ of 0.43 h ± 0.27 with very rapid clearance of 23.64l . $h^{-1}$ .$kg^{-1}$ ± 1.22. The time in which the plasma conc. of LX079 was higher than the in vitro MIC (T > MIC) was less than 1 hour after IV bolus administration of the substance at 12.5 mg/kg. Following the oral administration at 12.5 and 25 mg/kg, the oral bioavailability was 27.583 ± 2.457 and 32.662 ± 7.715 respectively and the whole concentration-time curves were below the level of the in vitro MIC. These findings were correlated with the low stability (23.14 % ± 2.01) of the compound when incubated with mice hepatic microsomal enzymes. LX079 exhibited very short in vitro$t_{1/2}$ of 5.94 ± 2.36min with very high hepatic intrinsic clearance of 221.81± 88.23 (ml .$min^{-1}$/ standard mouse weight)when scaled from the in vitro data. Extensive first pass effect of the liver eventually leads to poor bioavailability and short $t_{1/2}$.
Highly interesting was the observation that Lx079 accumulated in macrophages to levels which were able to control the growth of mycobacteria showing its potential to be used as a mycobacterial drug candidate. This was further supported by effectivity experiments in mice where clear positive inhibitory effect on the survival of the TB pathogen was observed with increasing dose of Lx079. Surprisingly, the maximum dose of Lx079 used was approximately three times less than

the current drug INH which is routinely used for the treatment of tuberculosis. In the light of highly favourable toxicity profile of Lx079, it is highly expected that higher doses with tolerable toxicity shall even prove more effective than INH.

References:

Agren, D., et al., (2008) J MolBiol, 377(4),1161-1173.
Balganesh, T. S., et al., (2004). Current Science, 86(1), 167-176.
Changsen, C., et al., (2003), 47(12), 3682-3687.
Corbett, E. L., et al., (2003), Arch Intern Med, 163, 1009-1021.
Davies, B. and Morris, T. (1993), Pharmaceutical Research, 10(7), 1093-1095.
Fenhalls, G., et al., (2002), Infect Immun, 70(11), 6330-6338.
Geraldes Santos, M. D. L. S., et al., (2007), Revistalatino-americana de enfermagem, 15 Spec No, 762-767.
Haydel, S. E. (2010), Pharmaceuticals (Basel, Switzerland), 3(7), 2268-2290.
Houston, J. B. (1994), Biochemical Pharmacology, 47(9), 1469-1479.
Koul, A., et al., (2011), Nature, 469, 483-490.
Lee, R. E., et al., (2003), Journal of Combinatorial Chemistry, 5(2), 172-187.
Marriner, G. A., et al., (2011), Top Med Chem, 7, 47-124.
Naritomi, Y., et al.,(2001),Drug MetabDispos.,29(10),1316-24.
Nassar, A. F., et al., (2009), Drug metabolism handbook: concepts and applications. John Wiley and Sons.
Nuermberger, E. L., et al., (2010), Respirology, 15(5), 764-778.
Palomino, J., et al., (2002), Antimicrobial Agents and Chemotherapy.46(8), 2720-2722.
Rowland, K. (2012), Nature News, (13 January).
Sacchettini, J. C., et al.,(2008), Nature Reviews. Microbiology, 6(1), 41-52.
Sánchez, J. G. B., Kouznetsov, V. V. (2010), Brazilian Journal of Microbiology, 41 (2), 270-277.
Shahab, F.M.,et al., (2010), Xenobiotica , 40 (3), 225-234.
WHO.(2007). Tuberculosis facts.
www.admescope.com
www.cyprotex.com
Zapol'skii, V. A.; et al., Synlett 2007, 10, 1507-1512.
V. A. Zapol'skii, et al., Z. Naturforsch. 2010, 65b, 843 - 860.
V. A. Zapol'skii, et. al., Beilstein J. Org. Chem. 2012, 8, 621-628.


## Claims

1. Compounds according to the general formula III or salts thereof

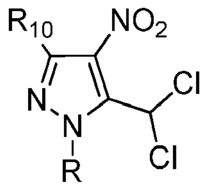

(III)

wherein
R represents in each case an optionally substituted aryl group or an optionally substituted heteroaryl group, preferably, R represents one of the radicals

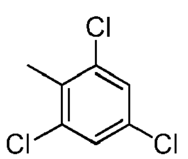

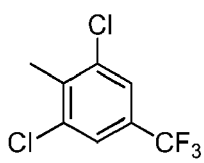

$R_{10}$ is selected from

i) $X_1$-$CR_{11})_2)_n$-$X_2$-
with $X_2$ being N or O;
$X_1$ is either $R_5R_6N$- or $R_5O$-,
$R_5$ is H, an optionally substituted, straight or branched $C_1$-$C_{10}$ alkyl group or an optionally substituted $C_3$-$C_8$ cycloalkyl group, or unsubstituted or substituted aryl, like benzyl, an optionally substituted hereroaryl group, preferably one of the radicals of a methyl, ethyl or isopropyl group;
$R_6$ is H, or an acyl group, or an optionally substituted straight or branched $C_1$-$C_{10}$ alkyl group, or an unsubstituted or substituted aryl group, like benzyl group, or an optionally substituted heteroaryl group;
n is an integer of 1 to 5, like 1, 2, or 3, and
$R_{11}$ is independently from each other H, or an optionally substituted straight or branched $C_1$-$C_{10}$ alkyl group, or an optionally substituted $C_3$-$C_8$ cycloalkyl group, an optionally substituted aryl group, or an ptionally substituted heteroaryl group;
ii.) a heterocyclic group, like a heteroaryl group, whereby said group may be substituted, and whereby said groups is linked via a heteroatom present in the heterocyclic group, like a heteroaryl group, with the pyrazol ring; or
iii.) $R_{12}R_{13}N$-
with $R_{12}$ and $R_{13}$ being independently from each other H, or an optionally substituted straight or branched $C_1$-$C_{10}$ alkyl group, an optionally substituted $C_3$-$C_8$ cycloalkyl group, an optionally substituted aryl group, or an optionally substituted heterocyclic group;

or pharmaceutically acceptable salts thereof.

**2.** The compounds according to claim 1 being a compound of formula I or salts thereof according to formula II

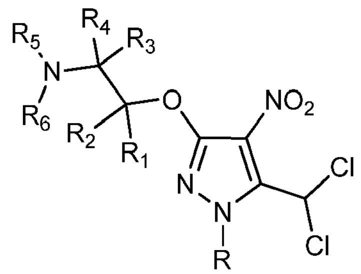

(I)

wherein R represents in each case an optionally substituted aryl group or an optionally substituted heteroaryl group, preferably, R represents one of the radicals

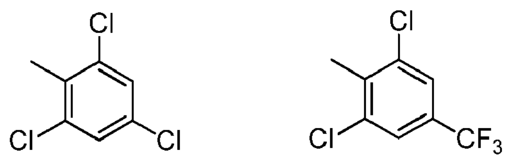

$R_1$-$R_4$ is independently from each other H, or an optionally substituted straight or branched $C_1$-$C_{10}$ alkyl group, or an optionally substituted $C_3$-$C_8$ cycloalkyl group, an optionally substituted aryl group, or an optionally substituted heteroaryl group;
$R_5$ is H, an optionally substituted straight or branched $C_1$-$C_{10}$ alkyl group or an optionally substituted $C_3$-$C_8$ cycloalkyl group, or unsubstituted or substituted aryl, like benzyl, or an optionally substituted heteroaryl, preferably, $R_5$ is one of the radicals of an H, methyl, ethyl or isopropyl group, $R_6$ is H, or an acyl group, or an optionally substituted straight or branched $C_1$-$C_{10}$ alkyl group, or an optionally substituted $C_3$-$C_8$ cycloalkyl group, or unsubstituted or substituted aryl group, like benzyl group, or an optionally substituted heteroaryl group;
or pharmaceutically acceptable salts (II) thereof,

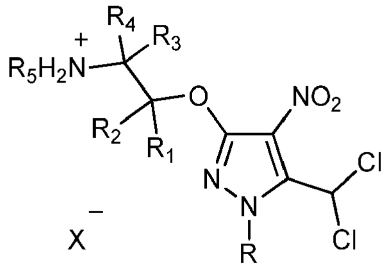

(II)

in which
$R_1$-$R_5$ are the same radicals as in formula (I)
$X^-$ is a suitable anion for forming the salt, preferably represents one of the anions $Cl^-$, $Br^-$, $BF_4^-$, $PF_6^-$, $SO_3H^-$, said compounds of formula I or II may be present in form of its solvates or hydrates.

3. The compounds according to claim 1 or 2, wherein any one of $R_1$ to $R_4$ are independently from one another hydrogen or a $C_1$-$C_4$ alkyl chain, in particular, wherein $R_1$ to $R_4$ are hydrogen.

4. The compounds according to any one of the preceding claims, wherein the residues $R_5$ and $R_6$ are independently from one another a straight or branched $C_1$-$C_4$ alkyl chain or hydrogen, in particular, hydrogen.

5. The compounds according to any one of the preceding claims, wherein R is a substituted aryl group or heteroaryl group, in particular, wherein R is

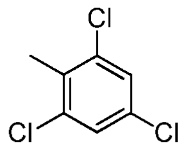 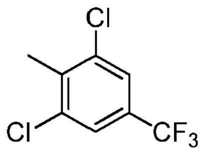.

6. The compounds according to any one of the preceding claims, wherein R is

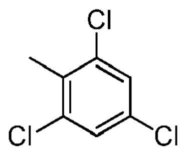 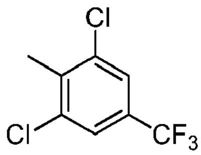,

and
$R_1$ to $R_6$ are independently from one another hydrogen and, if appropriate, $X^-$ is a halogen.

7. A pharmaceutical composition containing a compound according to any one of claims 1 to 6.

8. The compounds according to any one of claims 1 to 6 for use in the treatment of infectious diseases.

9. The compounds according to claim 8 for use in the treatment of bacterial infection.

10. The compounds according to claim 8 or 9 for use in the treatment of mycobacterial infection.

11. The compounds according to claim 10 for use in treating infection with mycobacterium tuberculosis, mycobacterium bovis, mycobacterium laprae or mycobacterium ulcerans.

12. The compounds according to any one of the preceding claims adapted for use for administration in form of a tablet.

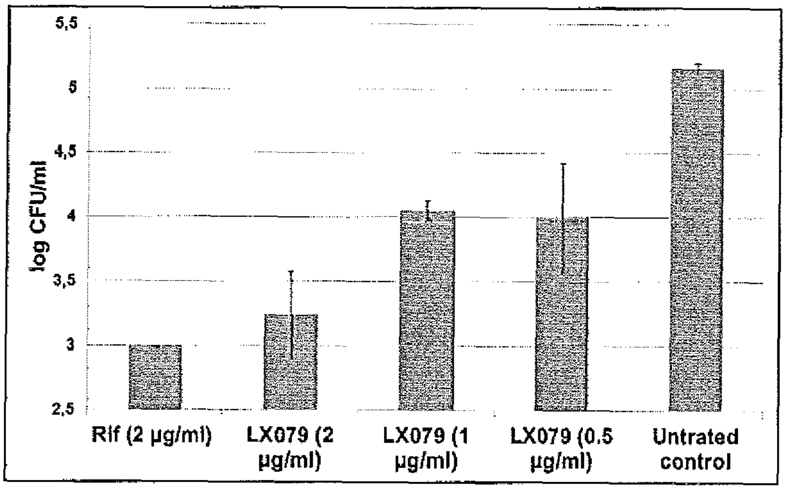


Figure 1

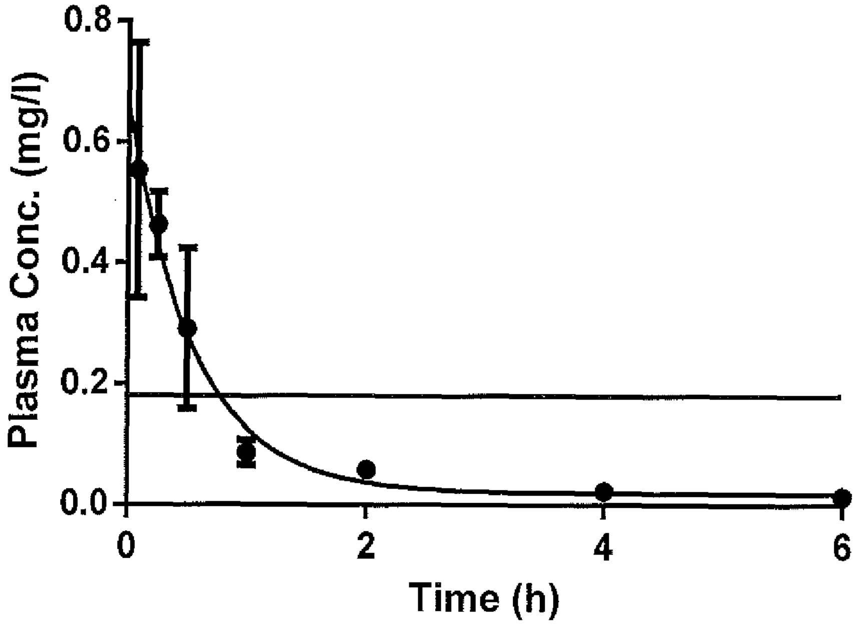


Figure 2

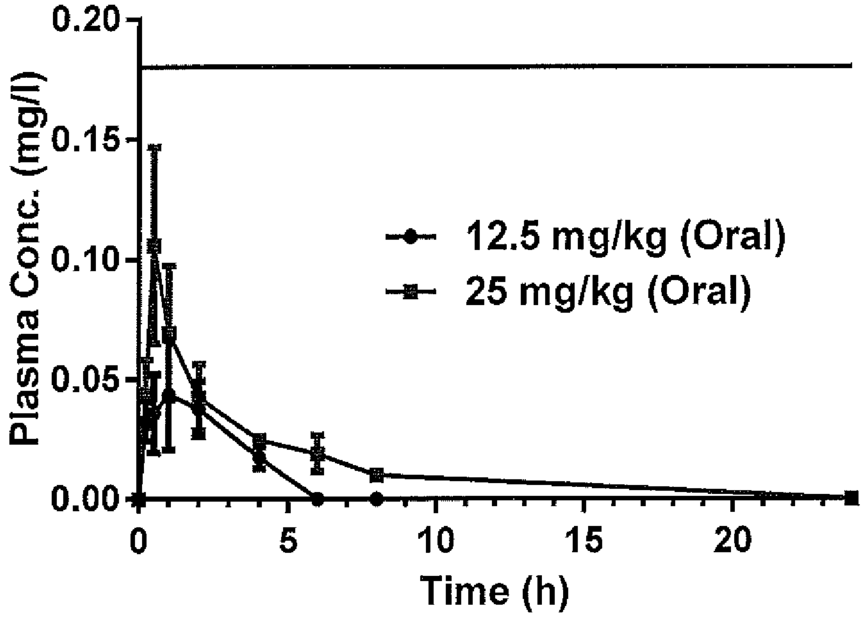


Figure 3

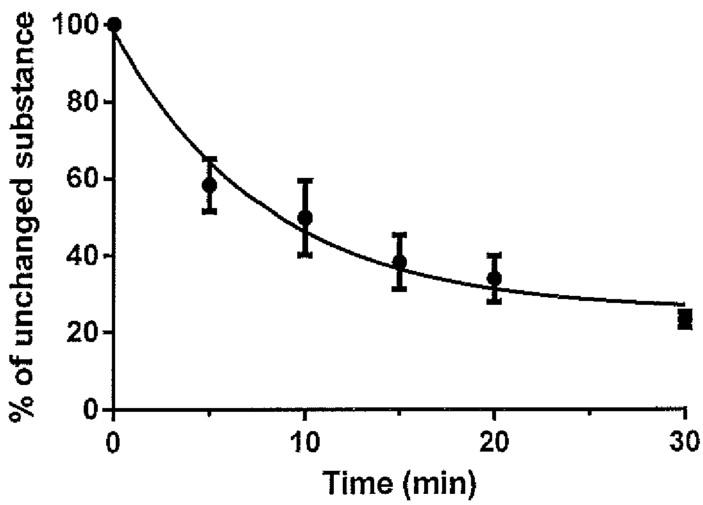


Figure 4

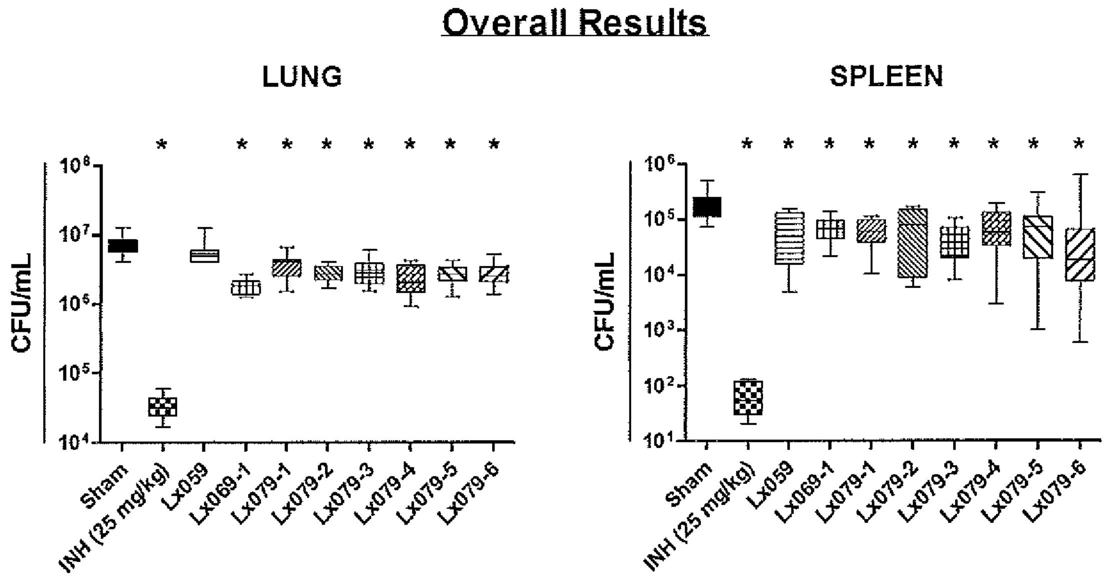


Figure 5

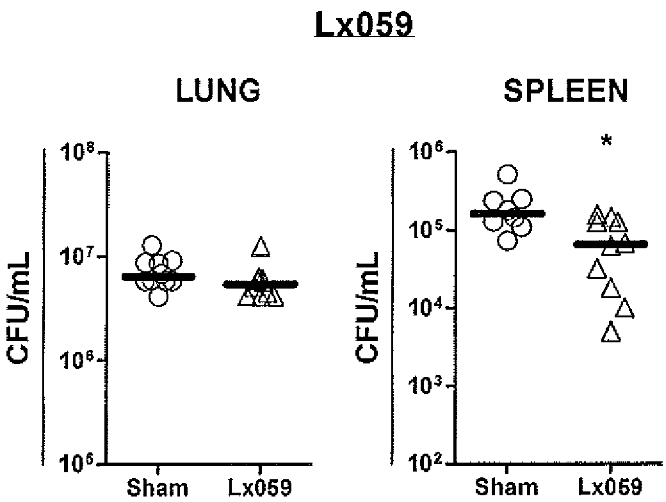


Figure 6

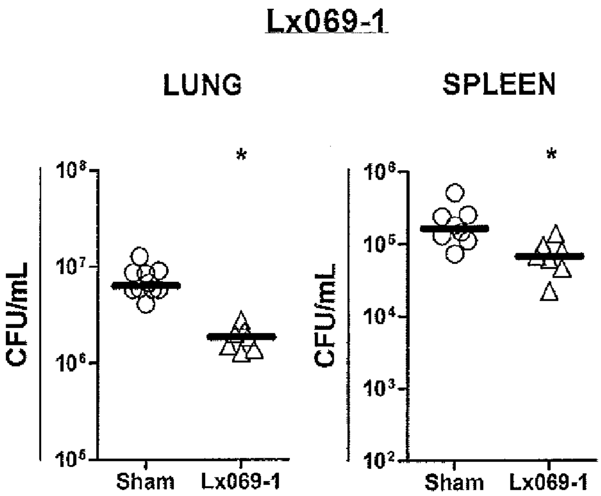


Figure 7

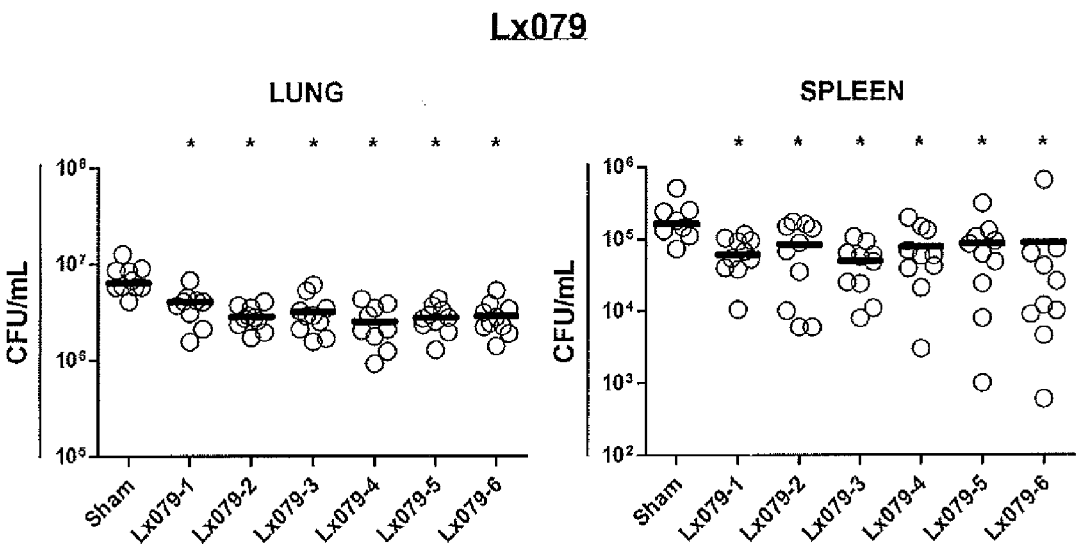


Figure 8

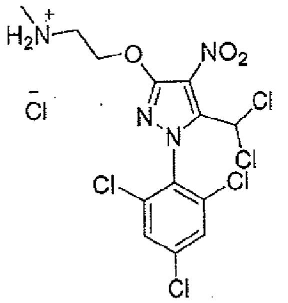

vz0825
Lx079

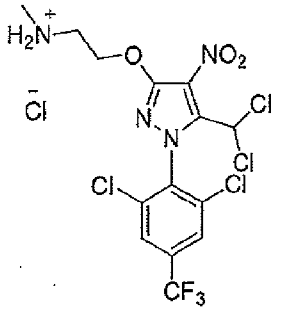

vz 2090

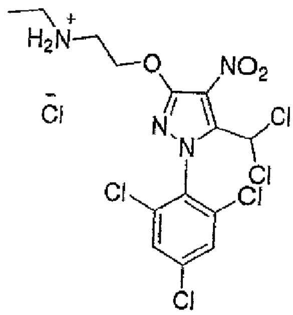

vz 2106

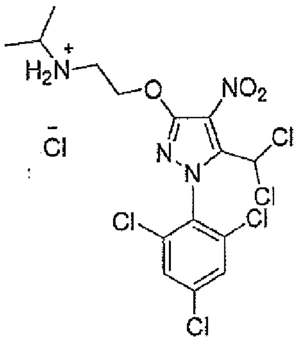

vz 2109

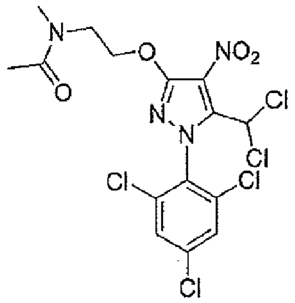

vz 2082

Fig 9a

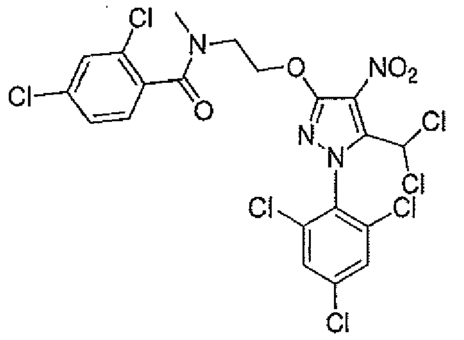

vz 2088

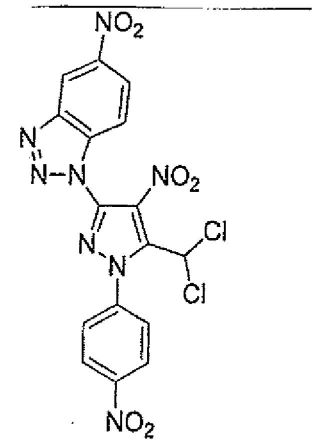

vz 0536

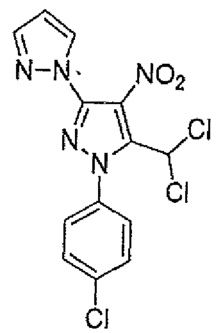

vz 1070

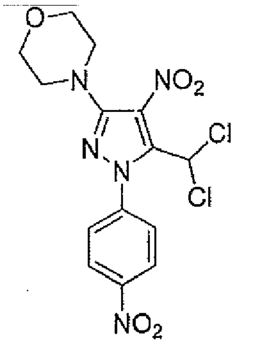

vz 1172

Fig 9b

European Patent Office

# EUROPEAN SEARCH REPORT

Application Number
EP 13 17 8220

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GB 783 706 A (MAY & BAKER LTD) 25 September 1957 (1957-09-25) * page 1, line 20 - page 1, line 28; claims 1,2; examples * | 1-12 | INV. C07D231/46 A61K31/4152 A61P31/06 |
| X | VIKTOR ZAPOL'SKII ET AL: "Chemistry of polyhalogenated nitrobutadienes, 10: Synthesis of highly functinalized heterocycles with a rigid 6-amino-3-azabicyclic[3.1.0]hexane moiety", BEILSTEIN JOURNAL OF ORGANIC CHEMISTRY, vol. 8, 23 March 2012 (2012-03-23), pages 821-828, XP009172499, * page 627, "conclusion"; compounds 27,28 * | 1,5,7 | |

TECHNICAL FIELDS SEARCHED (IPC)

C07D
A61K
A61P

The present search report has been drawn up for all claims

1

| Place of search | Date of completion of the search | Examiner |
|---|---|---|
| Munich | 13 September 2013 | Schmid, Arnold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 17 8220

This annex lists the patent family members relating to the patent documents cited in the above-mentioned European search report. The members are as contained in the European Patent Office EDP file on
The European Patent Office is in no way liable for these particulars which are merely given for the purpose of information.

13-09-2013

| Patent document cited in search report | Publication date | Patent family member(s) | Publication date |
|---|---|---|---|
| GB 783706 A | 25-09-1957 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. Mack Publishing Co, 1990
- **AGREN, D. et al.** *J MolBiol,* 2008, vol. 377 (4), 1161-1173
- **BALGANESH, T. S. et al.** *Current Science,* 2004, vol. 86 (1), 167-176
- **CORBETT, E. L. et al.** *Arch Intern Med,* 2003, vol. 163, 1009-1021
- **DAVIES, B. ; MORRIS, T.** *Pharmaceutical Research,* 1993, vol. 10 (7), 1093-1095
- **FENHALLS, G. et al.** *Infect Immun,* 2002, vol. 70 (11), 6330-6338
- **GERALDES SANTOS, M. D. L. S. et al.** *Revistalatino-americana de enfermagem, 15 Spec No,* 2007, 762-767
- **HAYDEL, S. E.** *Pharmaceuticals (Basel, Switzerland,* 2010, vol. 3 (7), 2268-2290
- **HOUSTON, J. B.** *Biochemical Pharmacology,* 1994, vol. 47 (9), 1469-1479
- **KOUL, A. et al.** *Nature,* 2011, vol. 469, 483-490
- **LEE, R. E. et al.** *Journal of Combinatorial Chemistry,* 2003, vol. 5 (2), 172-187
- **MARRINER, G. A. et al.** *Top Med Chem,* 2011, vol. 7, 47-124
- **NARITOMI, Y. et al.** *Drug MetabDispos.,* 2001, vol. 29 (10), 1316-24
- **NASSAR, A. F. et al.** Drug metabolism handbook: concepts and applications. John Wiley and Sons, 2009
- **NUERMBERGER, E. L. et al.** *Respirology,* 2010, vol. 15 (5), 764-778
- **PALOMINO, J. et al.** *Antimicrobial Agents and Chemotherapy.,* 2002, vol. 46 (8), 2720-2722
- **ROWLAND, K.** *Nature News,* 13 January 2012
- **SACCHETTINI, J. C. et al.** *Nature Reviews. Microbiology,* 2008, vol. 6 (1), 41-52
- **SÁNCHEZ, J. G. B. ; KOUZNETSOV, V. V.** *Brazilian Journal of Microbiology,* 2010, vol. 41 (2), 270-277
- **SHAHAB, F.M.** *Xenobiotica,* 2010, vol. 40 (3), 225-234
- WHO. *Tuberculosis facts,* 2007
- **ZAPOL'SKII, V. A. et al.** *Synlett,* 2007, vol. 10, 1507-1512
- **V. A. ZAPOL'SKII et al.** *Z. Naturforsch.,* 2010, vol. 65b, 843-860
- **V. A. ZAPOL'SKII.** *Beilstein J. Org. Chem.,* 2012, vol. 8, 621-628